# EUROPEAN PATENT SPECIFICATION

(11) **EP 1 893 360 B1**
(45) Date of publication and mention of the grant of the patent: **04.08.2010**
(21) Application number: 06768908.3
(22) Date of filing: 16.06.2006
(51) Int. Cl.: B09B 3/00

(54) **CARCASS DISPOSER**
SCHLACHTKÖRPERENTSORGER
ÉLIMINATEUR DE CARCASSE

(30) Priority: 20.06.2005 KR 20050052868
(43) Date of publication of application: 05.03.2008
(73) Proprietor: Yemyeong Industry, Kongju National University, Budae-dong Cheonan-si Chungcheongnam-do 330-717 (KR)
(72) Inventor: MOON, Sung-Ho, Chungcheongnam-do 330-813 (KR)
(74) Representative: Hertz, Oliver
(86) International application number: PCT/KR2006/002317
(87) International publication number: WO 2006/137656

(56) References cited:
- KR-Y1- 200 203 183
- KR-Y1- 200 287 987
- US-A- 5 119 994

## Description

### Technical Field

The present invention relates to complete sterilization of any organic matter, such as dead livestock, livestock waste, carcass etc. by heating them with high-pressure steam for a long time, and more particularly to a carcass disposer, in which a steam drum and a horizontally-movable cover are integrally mounted to a base frame, and a carcass loading table is integrally coupled to the cover, and the carcass loading table is automatically and positively taken in or out when the cover is closed or opened, thereby providing easy working, eliminating separate construction for a highly flat floor, and providing a compact structure and decrease in an occupation area for installation to thus improve the capability of utilizing a space.

### Background Art

In general, waste livestock which are infected with various diseases or contagious diseases and are dead in group, livestock byproducts discharged from livestock factories in quantities, and the like have been buried or reused as feedstuffs for breeding other animals. However, the burial of the waste livestock has been legally prohibited, and furthermore, there has been still a fear of secondary infection when the livestock that have been died of diseases or contagious diseases are fed to other animals. In addition, due to an offensive odor and a possibility of environmental pollution, it has been difficult to dispose them sanitarily.

Fortunately, various attempts have been made to sanitarily dispose them recently.

As a result, there have been proposed Korean Utility Model Registration No. 203183, titled "APPARATUS FOR DISPOSING WASTE LIVESTOCK (hereinafter, referred to as "Reference 1"), Korean Utility Model Registration No. 302977, titled "APPARATUS FOR FORCEDLY DISCHARGING MOISTURE IN WASTE LIVESTOCK DISPOSER (hereinafter, referred to as "Reference 2"), and Korean Utility Model Registration No. 309968, titled "DOUBLE JACKET TYPE CARCASS DISPOSER" (hereinafter, referred to as "Reference 3"), the latter two of which have been filed by the same applicant of the present invention and issued to the same applicant of the present invention.

According to Reference 1, a steam drum is disposed on a quadrilateral frame and provided on one side thereof with a pivotable cover, which is selectively opened and closed in order to put waste livestock into or out of the steam drum.

Further, the waste livestock is placed on a transfer table, which is slidably disposed on a transfer frame. The transfer table is selectively guided to a transfer rail which is installed inside the steam drum, and enters the steam drum. The waste livestock can be sterilized in the steam drum by supplying high-pressure steam generated by operation of a boiler.

Meanwhile, in Reference 2, water vapor containing a large volume of moisture, i.e. humidified vapor, which is generated inside a steam drum, is forcibly sucked using an intake head, and then discharged outside to reduce an amount of residual moisture in the steam drum. Thereby, an amount of moisture permeating into waste livestock is small, so that the capability to dispose the waste livestock can be doubled.

Reference 3 is an improvement on Reference 2. In Reference 3, water vapor inside a steam drum is forcibly discharged. In addition, a double jacket having an inner space outside the steam drum (i.e. an outer chamber) is formed. Steam is supplied into the inner space, and thus the steam drum is heated by indirect heat of the inner space. Thereby, moisture is evaporated and removed in a short time. Further, a disposing speed of a target to be disposed can be remarkably improved.

However, the conventional arts leave more or less improvements in common in putting the waste livestock into the steam drum.

In other words, the steam drum is separated from the transfer frame moving the transfer table on which the waste livestock rests toward an opening of the steam drum. Hence, when a foundation work for a floor is not accurately carried out, the transfer frame and the transfer rail installed inside the steam drum cannot be accurately maintained on a horizontal plane. As a result, the transfer table cannot be smoothly delivered from the transfer frame to the transfer rail of the steam drum.

Therefore, in order to accurately maintain the transfer frame and the transfer rail of the steam drum in a horizontal state, the floor should be accurately constructed in the horizontal state. Accordingly, the resulting incidental construction is inevitably required.

Further, when the steam drum is not used, the transfer frame and the transfer table are left outside as they are, and thus rusted or corroded. Wastes remaining after the disposing work are exposed outside, which is very unsanitary.

When the constituents such as the transfer frame and the transfer table are included, not only the manufacturing cost increases but also the equipment size becomes increasingly larger.

Moreover, the cover that selectively opens and closes the steam drum is opened or closed while pivoting on an L-shaped pivot in one direction. Thus, only when a sufficient space according to a radius of rotation should be secured, smooth opening/closing is possible. Further, when the cover is opened or closed, the transfer table is located beyond a rotational space of the cover (i.e. at a position spaced apart from the steam drum by a predetermined distance). As such, a wide occupation area according to installation of the equipment should be secured.

In addition, the input operation of the waste livestock requires many work steps, such as the opening and closing of the cover, the transferring of the transfer frame, and the transferring of the transfer table. Thus, the operation is very complicated, and because the input work steps are not successive, the operation requires much time.

### Disclosure of Invention

### Technical Problem

Therefore, the present invention has been made in view of the above-mentioned problems, and it is an objective of the present invention to provide a carcass disposer, capable of automatically taking in or out of a carcass loading table when a slidable cover is closed or opened, inhibiting the occurrence of corrosion and simultaneously maintaining clean sanitation because the carcass loading table is located inside a steam drum when the carcass disposer is notused, and promoting reduction of manufacturing costs and maximization of the capability of utilizing a space because an overall structure is made compact and simple.

### Technical Solution

According to an aspect of the present invention, there is provided a carcass disposer, which is structured in such a manner that a steam drum and a slidable cover are provided on both sides of a base frame, the steam drum and the cover are positioned on a same axis at all times, and a carcass loading table is integrally connected to an inner surface of the cover so that the carcass loading table is automatically taken in or out of the steam drum in cooperation with the cover as the cover is selectively closed and opened.

### Advantageous Effects

Because the steam drum and the slidable cover are integrated at both sides of the base frame, it is not necessary to construct a highly flat floor at a place where the carcass disposer is installed, so that incidental expenses can be saved.

Further, because the carcass loading table is integrally connected to the cover, the carcass loading table is automatically taken in or out of the steam drum as the cover is closed or opened. As a result, a carcass disposal work is very easy, simple, and continuous, so that it is possible to remarkably improve efficiency of the disposal work.

Also, when the carcass disposal work is not carried out, the carcass loading table is located inside the steam drum without being exposed outside, so that it is possible to inhibit the occurrence of corrosion and damage, as well as to maintain cleanness and sanitation.

Moreover, because the overall structure is very simple and compact, the manufacturing cost is saved, and simultaneously the occupied space accompanied with installation is small, so that the capability of utilizing the space is very good.

### Brief Description of the Drawings

The foregoing and other objects, features and advantages of the present invention will become more apparent from the following detailed description when taken in conjunction with the accompanying drawings in which:

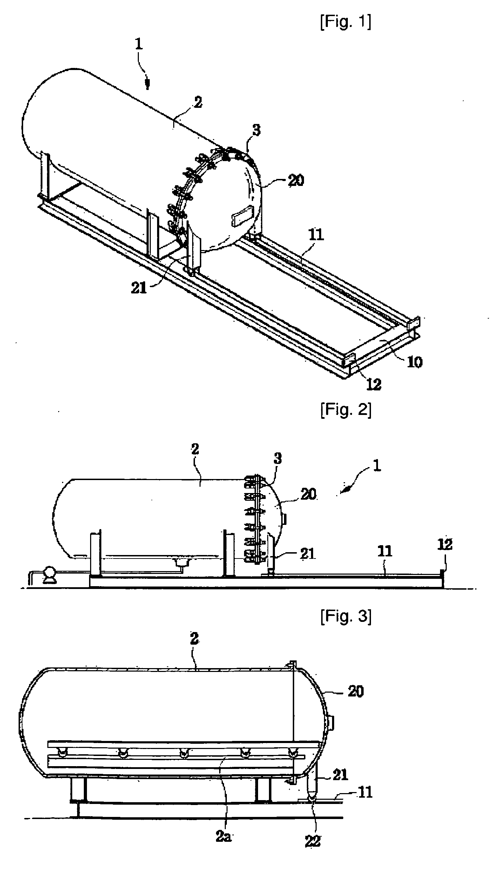

FIG. 1 is a perspective view of a carcass disposer according to the present invention;

FIG. 2 is a front view of a carcass disposer according to the present invention;

FIG. 3 is a vertical sectional view of a carcass disposer according to the present invention;

FIG. 4 is a vertical sectional view illustrating a state where a carcass loading table is taken in a steam drum according to the present invention;

FIG. 5 is a vertical sectional view illustrating a state where a cover is closed or opened according to the present invention;

FIG. 6 is a side sectional view illustrating a state where a cover is closed or opened according to the present invention;

FIG. 7 is a horizontal sectional view illustrating a state where a carcass loading table is installed according to the present invention;

FIG. 8 is an enlarged sectional view illustrating a state where a carcass loading table is coupled to a cover according to the present invention;

FIG. 9 is a perspective view illustrating an embodiment of a carcass loading table according to the present invention; and

FIG. 10 is a perspective view illustrating an embodiment of a carcass loading table according to the present invention.

<Explanation on essential elements of drawings>

2: steam drum 2a,11: transfer rails

10: base frame 20: cover

21: Cover supports 25: guide pins

30: carcass loading table 33: loading table brackets

36: loading flat plate 38: foldable flat plate

### Best Mode for Carrying Out the Invention

Now, a construction of the carcass disposer according to a preferred embodiment for effectively accomplishing the objectives of the present invention will be explained in detail with reference to attached drawings.

First, as for an essential construction of a carcass disposer 1 of the present invention, a steam drum 2 is disposed on one side of an upper portion of a base frame 10, and when a carcass is put into the steam drum 2, high-pressure steam is supplied into the steam drum 2 so as to sterilize the carcass.

In the carcass disposer 1 structured as above, a cover 20 is installed upright to cover the steam drum 2, and is silidably and horizontally moved along transfer rails 11 positioned on the upper portion of the other side of the base frame 10. A carcass loading table 30 is installed at the position perpendicular to the cover 20, with integrally connected to an inner surface of the cover 20. Thus, when the cover 20 is selectively opened or closed, the carcass loading table 30 is moved along transfer rails 2a inside the steam drum 2 along with the cover 20, so that the carcass loading table 30 is in and out of the steam drum 2 according to the movement of the cover 20.

Hereinafter, reference will be made in detail to the present invention having the schematic construction as above.

The base frame 10 is formed at a wider width over the conventional one without limitation to the construction in which the steam drum 2 is simply disposed thereon, and provides a transfer path and an installation space along and in which the cover 20 can slide horizontally on the other side of the steam drum 2. The transfer rails 11 are integrally attached to both longitudinal sides of the base frame 10 respectively, and stoppers 12 are installed at both leading ends of the transfer rails 11 respectively in order to prevent the cover 20 from being excessively moved to deviate from the transfer rails 11.

Cover supports 21 are respectively attached vertically to both sides of an outer lower surface of the cover 20 moving horizontally along the transfer rails 11. Transfer pulleys 22 are installed on respective lower ends of the cover supports 21, with connected to the transfer rails 11 installed on the base frame 10.

Thus, the cover 20 slidably and horizontally moves along the transfer rails 11, so as to hermetically close or open the steam drum 2 in a selective way.

Here, the steam drum 2 and the cover 20 can be coupled using fixing bolts 3, which are pivotably coupled to the steam drum 2, with flanges 24 passed through together, so that the steam drum 2 and the cover 20 maintain a firmly fastened state as a known construction.

Meanwhile, the carcass loading table 30, which is integrally connected to the inner surface of the cover 20, makes more simple a construction of means for putting the carcass into the steam drum 2, and simultaneously is connected to transfer rails 2a installed inside the steam drum 2, thereby serving to support the cover 20 so that the cover 20 can firmly maintain an upright state without falling down in either direction.

The carcass loading table 30 can be automatically taken in or out of the steam drum 2 while riding the transfer rails 2a installed inside the steam drum 2 and selectively moving together with the cover 20, so that it can provide rapidity, continuity, and easiness for the disposal work of a carcass.

With this construction, loading table brackets 33 are integrally attached to both sides of the inner surface of the cover 20, and formed with a plurality of through-holes 32 through which fasteners 31 are coupled. Nuts 34 are fastened to the fasteners 31 passing through the through-holes 32 of the loading table brackets 33, so that the carcass loading table 30 can maintain a firmly coupled state.

At this time, the through-holes 32 are preferably formed with a relatively greater diameter than that of the fasteners 31 so as to secure a constant distance between the carcass loading table 30 and the loading table brackets 33 after a fastening work. This is directed to allow the carcass loading table 30 to smoothly move along the transfer rails 2a installed in the steam drum 2 without fluctuation, although the cover 20 slightly fluctuates up and down due to the distance when foreign materials are adhered to the transfer rails 11 installed to the base frame 10 or a minute machining error accompanied with machining of the transfer rails 11 takes place.

The carcass loading table 30 is provided on both sides of the lower portion thereof with a plurality of pulleys 35, which are allowed to smoothly move along the transfer rails 2a installed to the steam drum 2. A loading flat plate 36 on which a carcass is placed is integrally formed on an upper surface of the carcass loading table 30.

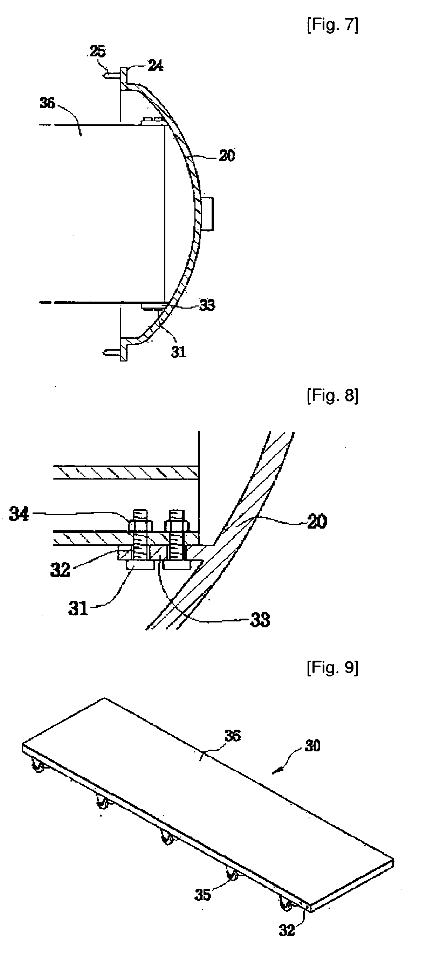

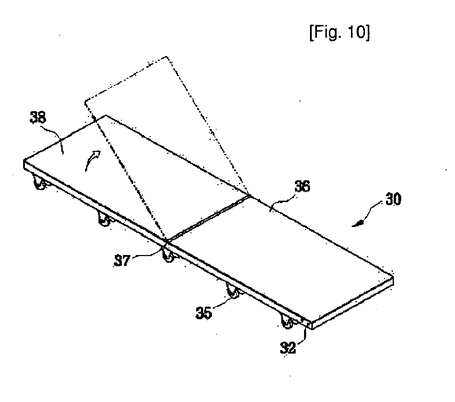

In this manner, the carcass loading table 30 allows the loading flat plate 36 to be installed throughout the upper surface thereof as in FIG. 9. Alternatively, as in FIG. 10, the carcass loading table 30 may be provided with a foldable flat plate 38, which is selectively opened while pivoting on a hinge shaft 37 on one side (i.e. on a lower surface) thereof.

The foldable flat plate 38 is opened to temporarily provide an exit so as for a worker to easily enter the steam drum 2 when the worker cleans the inside of the steam drum 2 after the carcass loading table 30 is taken outside.

Therefore, the worker can clean the inside of the steam drum 2 in a more convenient way.

Meanwhile, the present invention is adapted to guide the cover 20 to be accurately engaged with the steam drum 2 when the cover 20 is pushed toward the steam drum 2 and then closed hermetically. To this end, a plurality of guide pins 25 are radially provided on an inner surface of the flange 24 formed on the cover 20. Further, a plurality of guide holes 26 into which the plurality of guide pins 25 are inserted are formed on the flange 24 of the steam drum 2 corresponding to the cover 20.

Now, a process of disposing the carcass using the carcass disposer having the construction described above according to the present invention will be described.

First, the carcass loading table 30 of the present invention is very sanitary because it is located inside the steam drum 2 with the cover 20 closed hermetically. After the fixing bolts 3 maintaining the hermetically closed state of the cover 20 are loosened, the cover 20 is pulled backward. Then, the cover 20 is moved toward the direction opposite to the steam drum 2 along the transfer rails 11, and simultaneously the carcass loading table 30 integrally connected to the cover 20 is taken out of the steam drum 2 in cooperation with the cover 20 along the transfer rails 2a installed to the steam drum 2.

In other words, the carcass loading table 30 is taken in or out at the same time as the cover 20 is closed or opened, so that the working process is very simple, and the overall structure can be simplified.

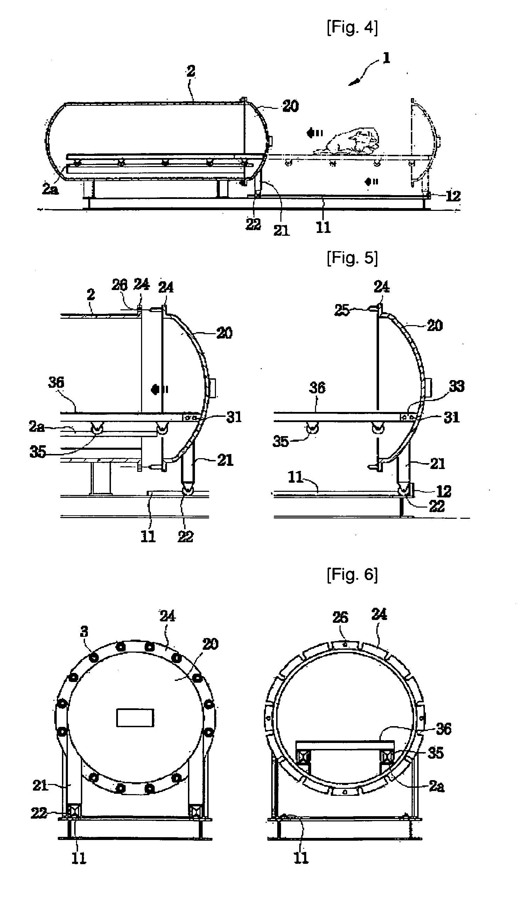

As described above, the cover 20 withdrawn outside maintains a stable state because one side of the carcass loading table 30 is seated on the transfer rails 2a of the steam drum 2 as in FIG. 4.

Next, after the carcass to be disposed is loaded on the carcass loading table 30 withdrawn outside, the cover 20 is pushed toward the steam drum 2. Then, the cover 20 and the carcass loading table 30 are smoothly and horizontally moved along the transfer rails 11 and 2a respectively. Thereby, the carcass loading table 30 enters the steam drum 2.

Consequently, the present invention provides the convenience capable of taking in or out the carcass loading table 30 by means of single manipulation of opening/closing the cover 20.

## Claims

1. A carcass disposer for sterilizing a carcass by supplying high-pressure steam into a steam drum (2) seated on one side of an upper portion of a base frame (10), the carcass disposer being **characterized in that** it comprises:
a cover (20) installed upright to horizontally move along transfer rails (11) installed on the other side of the base frame (10), and selectively opening and closing the steam drum (2); and
a carcass loading table (30) integrally connected to an inner surface of the cover (20), and being automatically taken in or out of the steam drum (2) along transfer rails (2a) inside the steam drum (2) as the cover (20) is selectively opened and closed.

2. The carcass disposer according to claim 1, **characterized in that** the cover (20) has a pair of cover supports (21) integrally connected to both sides of a lower portion thereof, and the cover supports (21) are provided on lower ends thereof with transfer pulleys (22) connected to the transfer rails (11) installed on both sides of the base frame (10) respectively.

3. The carcass disposer according to claim 1, **characterized in that** the carcass loading table (30) is coupled to loading table brackets (33) attached to both sides of the inner surface of the cover (20) by fasteners (31) passing through the loading table brackets (33); provided on both sides of a lower portion thereof with a plurality of pulleys (35) coupled to the transfer rails (2a) installed to the steam drum 2; and has a loading flat plate (36) on which the carcass is placed and which is integrally formed on an upper surface of thereof.

4. The carcass disposer according to claim 1, **characterized in that** the carcass loading table (30) is coupled to loading table brackets (33) attached on both sides of the inner surface of the cover (20) by fasteners (31) passing through the loading table brackets (33); provided on both sides of a lower portion thereof with a plurality of pulleys (35) coupled to the transfer rails (2a) installed to the steam drum 2; has a loading flat plate (36) on which the carcass is placed and which is integrally formed on an upper surface of thereof; and has a foldable flat plate (38) on one side of the loading flat plate (36) so as to be selectively opened and coupled pivotably.

5. The carcass disposer according to claim 3 or 4, **characterized in that** the loading table brackets (33) are formed with a plurality of through-holes (32) through which the fasteners (31) are coupled, and each of the through-holes (32) is formed at a greater diameter than that of each fastener (31) thereby to allow the carcass loading table (30) to have a constant distance.

6. The carcass disposer according to any one of claim 1 through 4, **characterized in that** the cover 20 is provided with a flange (24) having a plurality of guide pins 25 formed radially so as to guide the cover (20) to be accurately coupled with the steam drum (2) when the cover (20) is hermetically closed, and the plurality of guide pins (25) are inserted into a plurality of guide holes (26) formed on a flange (24) of the steam drum (2) corresponding to the cover (20).

## Patentansprüche

1. Kadaverentsorger zum Sterilisieren eines Kadavers mittels Zufuhr eines Hochdruckdampfs in einen Dampfzylinder (2), angeordnet an einer Seite eines oberen Abschnitts eines Basisrahmens (10), wobei der Kadaverentsorger **dadurch gekennzeichnet ist, dass** er umfasst
eine Abdeckung (20), angebracht aufrecht, um sich horizontal entlang Transferschienen (11) zu bewegen, angebracht an der anderen Seite des Basisrahmens (10) und wahlweise öffnend und schließend den Dampfzylinder (2); und
einen Kadaverbeladetisch (30), der integral verbunden ist mit einer inneren Fläche der Abdeckung (20), und der automatisch in den Dampfzylinder (2) gelangt oder aus dem Dampfzylinder (2) gelangt entlang Transferschienen (2a) in dem Dampfzylinder (2), wenn die Abdeckung (20) wahlweise geöffnet und geschlossen wird.

2. Kadaverentsorger gemäß Anspruch 1, **dadurch gekennzeichnet, dass** die Abdeckung (20) ein Paar Abdeckstützungen (21) aufweist, integral verbunden mit beiden Seiten eines unteren Abschnitts davon, und die Abdeckstützungen (21) bereitgestellt sind an unteren Enden davon mit Transferrollen (22), verbunden mit den Transferschienen (11), jeweils angebracht an beiden Seiten des Basisrahmens (10).

3. Kadaverentsorger gemäß Anspruch 1, **dadurch gekennzeichnet, dass** der Kadaverbeladetisch (30) an Beladetischlager (33) gekoppelt ist, befestigt an beiden Seiten der inneren Fläche der Abdeckung (20) mittels Befestigungsmitteln (31), tretend durch die Beladetischlager (33); bereitgestellt an beiden Seiten eines unteren Abschnitts davon mit einer Vielzahl von Rollen (35), gekoppelt an die Transferschienen (2a), angebracht an dem Dampfzylinder (2); und aufweisend eine flache Beladungsplatte (36), auf der der Kadaver platziert wird und die integral ausgebildet ist an einer oberen Fläche davon.

4. Kadaverentsorger gemäß Anspruch 1, **dadurch gekennzeichnet, dass** der Kadaverbeladetisch (30) an Beladetischlager (33) gekoppelt ist, befestigt an beiden Seiten der inneren Fläche der Abdeckung (20) mittels Befestigungsmitteln (31), tretend durch die Beladetischlager (33); bereitgestellt an beiden Seiten eines unteren Abschnitts davon mit einer Vielzahl von Rollen (35), gekoppelt an die Transferschienen (2a), angebracht an dem Dampfzylinder (2); aufweisend eine flache Beladeplatte (36), auf der der Kadaver platziert wird und die integral ausgebildet ist an einer oberen Fläche davon; und aufweisend eine klappbare flache Platte (38) an einer Seite der flachen Beladeplatte (36), um wahlweise öffenbar und koppelbar auf schwenkbare Weise zu sein.

5. Kadaverentsorger gemäß Anspruch 3 oder 4, **dadurch gekennzeichnet, dass** die Beladetischlager (33) ausgebildet sind mit einer Vielzahl von Durchgangslöchern (32), durch die die Befestigungsmittel (31) gekoppelt werden, und jedes der Durchgangslöcher (32) ausgebildet ist an einem größeren Durchmesser als der von jedem Befestigungsmittel (31), wodurch dem Kadaverbeladetisch (30) ermöglicht wird, eine konstante Distanz aufzuweisen.

6. Kadaverentsorger gemäß einem der Ansprüche 1-4, **dadurch gekennzeichnet, dass** die Abdeckung (20) bereitgestellt ist mit einem Flansch (24), aufweisend eine Vielzahl von Führungsstiften (25), radial ausgebildet, um die Abdeckung (20) zu führen, um akkurat mit dem Dampfzylinder (2) gekoppelt zu werden, wenn die Abdeckung (20) hermetisch geschlossen wird, und die Vielzahl der Führungsstifte (25) eingeführt werden in eine Vielzahl von Führungslöchern (26), ausgebildet an einem Flansch (24) des Dampfzylinders (2) entsprechend der Abdeckung (20).

## Revendications

1. Éliminateur de carcasse pour stériliser une carcasse en alimentant de la vapeur d'eau à haute pression dans un collecteur de vapeur (2) installé d'un côté d'une partie supérieure d'un châssis de base (10), l'éliminateur de carcasse étant **caractérisé en ce qu'**il comprend :
un couvercle (20) installé droit pour se déplacer horizontalement le long de rails de transfert (11) installés de l'autre côté du châssis de base (10), et ouvrir et fermer sélectivement le collecteur de vapeur (2) ; et
une table de chargement de carcasse (30) raccordée de manière solidaire à une surface interne du couvercle (20) et entrant et sortant automatiquement du collecteur de vapeur (2) le long des rails de transfert (2a) à l'intérieur du collecteur de vapeur (2) lorsque le couvercle (20) est sélectivement ouvert et fermé.

2. Éliminateur de carcasse selon la revendication 1, **caractérisé en ce que** le couvercle (20) a une paire de supports de couvercle (21) raccordés de manière solidaire aux deux côtés de leur partie inférieure, et les supports de couvercle (21) sont prévus sur leurs extrémités inférieures avec des poulies de transfert (22) raccordées aux rails de transfert (11) installés des deux côtés du châssis de base (10) respectivement.

3. Éliminateur de carcasse selon la revendication 1, **caractérisé en ce que** la table de chargement de carcasse (30) est couplée à des supports de table de chargement (33) fixés des deux côtés de la surface interne du couvercle (20) par des fixations (31) qui passent par les supports de table de chargement (33), prévus des deux côtés de leur partie inférieure avec une pluralité de poulies (35) couplées aux rails de transfert (2a) installés sur le collecteur de vapeur (2) ; et a une plaque plate de chargement (36) sur laquelle la carcasse est placée et qui est formée de manière solidaire sur sa surface supérieure.

4. Éliminateur de carcasse selon la revendication 1, **caractérisé en ce que** la table de chargement de carcasse (30) est couplée aux supports de table de chargement (33) fixés des deux côtés de la surface interne du couvercle (20) par des fixations (31) passant par les supports de table de chargement (33), prévus des deux côtés de sa partie inférieure avec une pluralité de poulies (35) couplées aux rails de transfert (2a) installés sur le collecteur de vapeur (2) ; a une plaque plate de chargement (36) sur laquelle la carcasse est placée et qui est formée de manière solidaire sur sa surface supérieure ; et a une plaque plate pliable (38) d'un côté de la plaque plate de chargement (36) afin d'être sélectivement ouverte et couplée de manière pivotante.

5. Éliminateur de carcasse selon la revendication 3 ou 4, **caractérisé en ce que** les supports de table de chargement (33) sont formés avec une pluralité de trous de passage (32) à travers lesquels les fixations (31) sont couplées, et chacun des trous de passage (32) est formé à un plus grand diamètre que celui de chaque fixation (31) pour permettre ainsi à la table de chargement de carcasse (30) d'avoir une distance constante.

6. Éliminateur de carcasse selon l'une quelconque des revendications 1 à 4, **caractérisé en ce que** le couvercle (20) est prévu avec un rebord (24) ayant une pluralité de broches de guidage (25) formées de manière radiale afin de guider le couvercle (20) pour être couplé précisément avec le collecteur de vapeur (2) lorsque le couvercle (20) est hermétiquement fermé, et la pluralité de broches de guidage (25) est insérée dans une pluralité de trous de guidage (26) formée sur un rebord (24) du collecteur de vapeur (2) correspondant au couvercle (20).
